# DEMANDE DE BREVET EUROPEEN

(11) **EP 4 385 404 A1**
(43) Date de publication de la demande: **19.06.2024**
(21) Numéro de dépôt: 23216623.1
(22) Date de dépôt: 14.12.2023
(51) Int. Cl.: A61B 5/0537, B01L 1/00, A61B 10/00, A61B 5/00, B01L 3/00, G01N 33/50

(54) **DISPOSITIF DE MESURE DE PARAMÈTRES BIOLOGIQUES OU CHIMIQUES RELATIFS À LA PEAU D'UN UTILISATEUR OU À L EFFICACITÉ DERMO- COSMÉTIQUE ET DERMATOLOGIQUE D'UNE FORMULE DE SOIN DE LA PEAU**

(30) Priorité: 15.12.2022 FR 2213459
(71) Demandeur: WB Technologies, 75006 Paris (FR)
(72) Inventeur: GENAIN, Gilles, 75006 Paris (FR); LE TANNEUR, Sophie, 75006 Paris (FR)
(74) Mandataire: August Debouzy

(57) **Abrégé**

Dispositif de mesure de paramètres biologiques ou chimiques relatifs à la peau d'un utilisateur ou à l'efficacité dermo- cosmétique et dermatologique d'une formule de soin de la peau comprenant :
un capteur configuré pour réaliser une mesure à partir d'une goutte d'une solution électrolyte comprenant des composés biologiques et/ou chimiques ou à partir d'une ouate imbibée d'une telle solution électrolyte,
un circuit électronique configuré pour recevoir des signaux électriques dudit capteur et pour réaliser un traitement en vue de transmettre des données de mesures à un dispositif distant,
un logement configuré pour recevoir ladite goutte et/ou un dispositif de prélèvement comprenant ladite ouate imbibée.

## Description

### Domaine technique

La présente invention concerne un dispositif de mesure de paramètres relatifs à la peau d'un utilisateur, ou à la mesure de l'efficacité d'ingrédients utilisés par les laboratoires cosmétiques pour la mise au point de produits de soin de la peau, et de formules pour de tels produits.

L'invention concerne en particulier la mesure objective, pertinente et rapide des paramètres de performance de certains actifs utilisés sur la peau qui peut être saine par exemple via des crèmes ou autres traitements dermo-cosmétiques ou dermatologiques.

L'invention a des applications relatives à la surveillance de la bonne santé de la peau en permettant des mesures sur des actifs et des ingrédients utilisés sur la peau ainsi que des mesures identiques du « *tubo »* au vitro et au « *vivo* », du laboratoire à la peau reconstruite, aux explants de peau et à la salle de bain, du personnel de laboratoire à l'utilisateur etc. L'invention a aussi des applications dans la surveillance de la bonne santé de la peau en l'absence de traitements cosmétiques ou dermo-cosmétiques et la détection de certaines pathologies cutanées. D'habitude, une mesure électro-chimique est faite soit in tubo, en mesurant un actif dilué, soit in vitro, sur une peau reconstruite, soit un vivo, sur un morceau de peau, rarement sur le visage pour des raisons de tenue de goutte.

### Arrière-plan technologique

La composition chimique de la peau présente un grand intérêt pour sa caractérisation. Par exemple, le pH de la surface de la peau apporte des informations complémentaires pertinentes aux données physiques. Un autre exemple est la teneur en facteurs naturels d'hydratation (dits « NMF », acronyme anglais de « *Natural Moisturizing Factors* », soit en français « *facteurs naturels d'hydratation* ») pour déterminer le niveau d'hydratation de la peau. Les NMFs aident à maintenir l'hydratation à l'intérieur des cornéocytes pour garder la couche cornée hydratée en liant les molécules d'eau. Des études ont démontré leur réduction soluble associée à la peau sèche ou aux individus atteints de dermatite atopique (parfois désignée sous l'acronyme DA). La méthode la plus fiable pour quantifier sélectivement les NMFs reste la méthode chromatographique, en particulier la chromatographie liquide à haute performance (parfois désignée sous l'acronyme HPLC). En outre, la capacité antioxydante de la peau est représentative de la capacité de la peau à se défendre ou à répondre à des agressions de l'environnement telle la capacité à résister aux rayons ultraviolets (UV, pollution). Enfin, l'effet barrière de la peau est une forme de synthèse de tous ces paramètres.

Pour la majorité des événements métaboliques dans la peau, de nombreux processus biologiques sont basés sur des échanges électroniques. L'électrochimie apparaît donc comme une technologie capable de qualifier et de quantifier de manière rapide et non invasive les réactions biologiques impliquées dans le fonctionnement de la peau. L'électrochimie permet de détecter tout composé ayant des propriétés antioxydantes si son composé d'affinité avec le capteur et le solvant est suffisant. Les antioxydants peuvent interagir directement, spontanément et immédiatement avec les espèces réactives de l'oxygène (ROS), responsables d'un stress oxydatif délétère. Leur présence prévient les dommages irréversibles causés à tout composé cellulaire et une teneur élevée en espèces antioxydantes a été associée à des niveaux de stress oxydatif plus faibles. Pour certaines espèces telles que la vitamine C/acide ascorbique, l'électrochimie a démontré avec succès la fiabilité de leur détection, avec un intérêt croissant pour le développement de son application pour la détection de biomarqueurs physiologiques pour surveiller les conditions de santé cutanée. Connue pour sa simplicité d'utilisation et sa rapidité de mesure, l'électrochimie permet également des mesures *in situ* ou *ex situ* non invasives, sans prétraitement important et fastidieux de l'échantillon. De plus, certains NMFs présentent des propriétés antioxydantes et sont donc susceptibles d'être détectés par les capteurs de manière spécifique.

Sur la base de ces connaissances, divers dispositifs ont été proposés pour déterminer un état de la peau d'un utilisateur de manière non invasive. Par exemple, le document US 6,108,570 divulgue un dispositif non-invasif pour déterminer la quantité d'oxydants et d'anti-oxydants utilisant des électrodes plongées dans une solution en contact avec la peau d'un utilisateur. Le document US 2021/0076988 divulgue un dispositif non-invasif pour mesurer le niveau de glucose d'un utilisateur via un une membrane adhésive posée sur la peau d'un utilisateur. Le document US 8,515,523 divulgue un dispositif pour mesure l'état « *redox* » de la peau d'un utilisateur (c'est-à-dire la balance entre les oxydants et les anti-oxydants de la peau) au moyen de microélectrodes. Le document WO 2004/030537 divulgue un dispositif de mesure de propriété physico-chimiques de la peau au moyen de micro-électrodes. Le document FR 3 042 872 divulgue un système de détermination de paramètres physico-chimiques et biologiques de la peau, comprenant un dispositif de nettoyage apte à recueillir des composés biologiques et/ou chimiques de la peau, un dispositif de mesure apte à déterminer les caractéristiques physiologiques de l'état de la peau, un dispositif d'analyse apte à analyser les caractéristiques des composés transférés sur le dispositif de nettoyage lors du nettoyage, et de façon liée, des moyens de calcul aptes à confronter ces caractéristiques avec des informations déclaratives fournies par l'utilisateur afin de déterminer des conseils dermo-cosmétiques.

Cependant, bien qu'il existe diverses solutions techniques utilisant l'électrochimie de la peau pour la caractériser, idem formules et actifs, il existe encore un besoin pour un dispositif répondant à plusieurs problématiques encore persistantes.

Parmi les besoins encore à satisfaire, on peut citer celui de la mesure identique tubo, vitro, vivo, des potentiels anti-oxydant, sébum, Natural Moisturizing Factors, avec une précision nécessaire et suffisante pour les concentrations usuelles dans l'univers dermo-cosmétique et dermatologiques, dans des environnements/matrices lipophiles et hydrophiles
On peut aussi citer le recueil de la perception de l'utilisateur et les facteurs extérieurs à cet utilisateur (pollution, exposome, bien-être, activités diverses).

En outre, la fiabilité de la prise de mesure reste un enjeu important, en fonction des variables telles que le temps de pose, le type de prélèvement et les processus en jeu.

D'autres besoins restent aussi à satisfaire. Il s'agit du caractère simple d'utilisation, portatif et connecté du dispositif. Il s'agit aussi de la conception adaptée aux nécessités environnementales, de recyclage et de réparabilité. Il s'agit enfin de la possibilité d'utiliser le dispositif en laboratoire, par un personnel expert et dans la vie privée par des utilisateurs non experts.

La présente invention s'inscrit dans ce cadre.

### Résume de l'invention

Selon un **premier aspect**, l'invention concerne un dispositif de mesure de paramètres biologiques ou chimiques relatifs à la peau d'un utilisateur ou à l'efficacité dermo- cosmétique et dermatologique d'une formule de soin de la peau comprenant :
un capteur configuré pour réaliser une mesure à partir d'une goutte d'une solution électrolyte comprenant des composés biologiques et/ou chimiques ou à partir d'une ouate imbibée d'une telle solution électrolyte,
un circuit électronique configuré pour recevoir des signaux électriques dudit capteur et pour réaliser un traitement en vue de transmettre des données de mesures à un dispositif distant,
un logement configuré pour recevoir ladite goutte et/ou un dispositif de prélèvement comprenant ladite ouate imbibée.

Par exemple, lesdits composés biologiques et/ou chimiques proviennent d'au moins l'un parmi la couche superficielle de la peau d'un utilisateur, de principes actifs ou de formules de soin de peau dermatologiques ou dermo- cosmétiques.

Par exemple, ladite mesure d'au moins l'un parmi la couche superficielle de la peau d'un utilisateur, de principes actifs ou de formules de soin de peau dermatologiques ou dermo- cosmétiques.

Par exemple encore, ledit circuit électronique est en outre configuré pour transmettre lesdites données de mesure audit dispositif distant.

Selon des réalisations, le dispositif comprend en outre une interface de connexion pour connecter ledit capteur audit circuit électronique, ladite connexion étant configurée pour connecter et déconnecter ledit capteur à la demande, de manière amovible.

Par exemple, ledit circuit électronique comporte un potentiostat, le potentiel dudit potentiostat étant analysé sous forme de rampe en escalier.

Par exemple, ledit capteur comporte au moins trois électrodes dont au moins l'une est formée d'un matériau choisi parmi le graphite, l'argent massif, le carbone vitreux et l'or.

Par exemple, le dispositif comprend trois électrodes et lesdites électrodes affleurent dans un même plan, relief possible de contact avec la ouate imbibée ou la goutte et sont distribuées selon les sommets d'un triangle isocèle dans ledit plan.

Selon un **deuxième aspect**, l'invention concerne un système de mesure de paramètres relatifs à la peau d'un utilisateur comprenant :
un dispositif selonle premier aspect de l'invention , et
un dispositif de prélèvement comprenant une ouate imbibée d'une solution électrolyte comprenant des composés biologiques et/ou chimiques.

Par exemple, lesdits composés biologiques et/ou chimiques proviennent d'au moins l'un parmi la couche superficielle de la peau d'un utilisateur ou de formules de soin de peau ou d'actifs dermatologiques ou dermo- cosmétiques.

Par exemple, le dispositif de prélèvement a une forme en poire, ladite ouate imbibée étant placée en son centre et un opercule recouvrant ladite ouate imbibée.

Par exemple, le système comporte un dispositif distant configuré pour recevoir des données de mesure dudit dispositif de prélèvement et pour réaliser au moins un traitement à partir de ces données.

Par exemple encore, le système comporte en outre au moins un serveur distant, configuré pour communiquer directement ou indirectement avec ledit dispositif distant et pour stocker des données d'utilisateur issues de mesures de paramètres relatifs à leur peau.

### Brève description des figures

D'autres caractéristiques et avantages de l'invention apparaîtront à la lecture de la présente description détaillée qui suit, à titre d'exemple non limitatif, et des figures annexées parmi lesquelles :
[Fig. 1] illustre un contexte d'implémentation de modes de réalisation de l'invention,
[Fig. 2] illustre un dispositif selon des modes de réalisation,
[Fig. 3] illustre un capteur selon des modes de réalisation,
[Fig. 4] illustre un capteur selon des modes de réalisation,
[Fig. 5] illustre un potentiel d'un potentiostat selon des modes de réalisation,
[Fig. 6] illustre une interface de connexion d'un capteur selon des modes de réalisation,
[Fig. 7] illustre un dispositif de prélèvement selon des modes de réalisation,
[Fig. 8] illustre un dispositif selon des modes de réalisation.

### Description détaillée de l'invention

Comme cela apparaitra à la lecture de la description détaillée, les modes de réalisation de l'invention peuvent se présenter sous la forme d'un boitier connecté intégrant un capteur avec au moins trois électrodes liées par un connecteur permettant de l'interfacer avec une électronique de mesure à base de potentiostat. Le boîter peut communiquer avec une application numérique permettant de gérer divers éléments. Il s'agit par exemple des questions d'agrément posées à l'utilisateur ainsi que la gestion d'un protocole de test (par exemple avec des temps de pose). Il s'agit aussi par exemple de l'analyse de signal généré par les marqueurs cutanés accumulés par le dispositif. Il s'agit encore par exemple des calculs qui découlent de cette analyse pour évaluer la performance des ingrédients ou formules appliquées sur la peau de l'utilisateur et le stockage dans une base de données. L'application numérique peut par ailleurs permettre d'anonymiser les données de l'utilisateur et relever les conditions atmosphériques (ultraviolets et pollution, GPS par exemple).

Pour relever des caractéristiques de bio-marqueurs de la peau, le dispositif peut interagir avec un patch pré-imbibé avec une solution électrolyte. Ce patch est appliqué sur la peau de l'utilisateur puis est inséré dans le dispositif pour analyse. Par exemple, la solution est une solution d'éthanol Selon d'autres réalisations, le dispositif réagit directement avec une goutte de solution électrolyte comprenant des composés biologiques et/ou chimiques provenant de couches superficielles de la peau de l'utilisateur ou des ingrédients actifs utilisés pour les formulations de soins dermo-cosmetiques

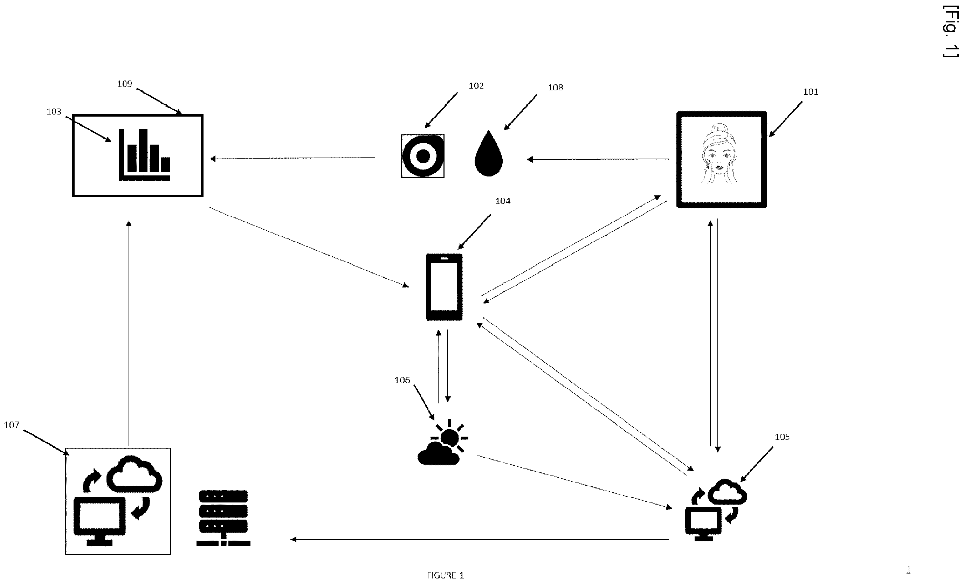

La **figure 1** illustre un contexte général d'implémentation de modes de réalisation de l'invention dans le cadre le plus complexe d'auto-administration du test par l'utilisateur final. Un utilisateur **101** réalise un auto-prélèvement d'un biomarqueur cutané **108** sur sa peau. Par exemple, il s'agit d'un marqueur NMF, d'un anti-oxydant, ou autre. Le biomarqueur est prélevé au moyen d'un patch **102** pré-imbibé d'une solution électrolyte posé sur la peau durant une certaine durée (par exemple, une minute).

Le patch ainsi porteur du biomarqueur est inséré dans un dispositif **109** selon des modes de réalisation. Ce dernier réalise ensuite une identification du biomarqueur. Une mesure est réalisée par électrolyse de réactions d'oxydoréduction via un capteur électrochimique. Ensuite, la nature des anti-oxydants est déterminée, ainsi que, par exemple, leur quantité et stabilité/réactivité. Cette analyse peut ensuite permettre de réaliser une détermination de l'effet barrière, capacité anti-ox. Il s'agit de déterminer une quantité d'antioxydants collectés par rapport à une valeur seuil en dessous de laquelle on peut considérer que l'effet barrière de la peau est rompu. Pour cette détermination, il est possible d'utiliser des abaques en fonction de l'âge de l'utilisateur, de son phototype ou autre.

Une fois l'ensemble des traitements réalisés par le dispositif, les données obtenues **103** peuvent faire l'objet de plusieurs utilisations. Les données issues de mesures de paramètres relatifs à la peau de plusieurs utilisateurs peuvent notamment être stockées dans des serveurs distants.

Le dispositif **109** peut communiquer ces données à un dispositif distant **104** mettant en oeuvre un procédé de traitement. Par exemple, le dispositif distant est un smartphone exécutant une application dédiée. La communication peut se faire sans fil, par exemple par le protocole Bluetooth. D'autres moyens de communication sont possibles. Le dispositif distant **104** peut par exemple réaliser tout traitement permettant de produire des courbes ou autres moyens de détection d'anti-oxydant ou autres biomarqueurs. Le traitement par le dispositif distant **104** peut aussi se faire au moyen de données **106** relatives aux conditions dans lesquels l'auto-prélèvement par l'utilisateur a été fait. L'analyse peut aussi avoir été faite en laboratoire. Ces données peuvent être générées par le dispositif distant ou reçues d'un autre dispositif dédié (non représenté).

Par ailleurs, le dispositif distant **104** peut aussi, au titre des traitements effectués, générer des questions relatives à la perception de l'utilisateur ou à sa typologie de peau (routine de soins ou autre). Ces questions sont ensuite présentées à l'utilisateur (ou un chercheur en laboratoire) et le dispositif distant recueille les réponses obtenues. Ces réponses pourront être associées aux résultats des traitements effectuées sur les données de mesure en vue d'un stockage dans une base de données.

Selon des réalisations, les traitements et calculs réalisés par le dispositif distant, le sont au moyen d'une intelligence artificielle. A cet effet, des données issues de divers utilisateurs (ou chercheurs) peuvent être collectées à des fins d'apprentissage de l'intelligence artificielle. A cet effet, le dispositif distant peut communiquer avec un premier serveur distant **105** qui va être chargé d'anonymiser les données. Selon des réalisations, ce serveur distant peut lui-même agréger les données **106** avec les données issues du dispositif distant **104.**

Une fois les données anonymisées, elles sont transmises à un deuxième serveur distant **107**, en charge de centraliser les données collectées par divers dispositifs distants et anonymisées par le premier serveur distant **105.**

Les modes de réalisation de l'invention offrent donc diverses fonctionnalités répondant aux besoins non satisfaits par les dispositifs de l'art antérieur. Il s'agit en particulier de la récupération de manière simple et efficace pour l'utilisateur (ou le chercheur) des caractéristiques de la peau et/ ou des actifs utilisés sur celle-ci. Il s'agit aussi de l'analyse rapide de ces caractéristiques et du traitement des données qui y sont relatives, au moyen notamment de données externes.

En outre, les moyens de réalisation de l'invention permettent de réaliser des prélèvements à tout moment, de manière non-invasive pour l'utilisateur (ou le chercheur). Les calculs peuvent être fait à la volée et les résultats peuvent être affichés à l'utilisateur (ou le chercheur) de manière rapide et fiable. Parmi les résultats qui peuvent être fournis à l'utilisateur (ou le chercheur), on peut citer la détection d'une dermatite atopique, une identification des anti-oxydants de la peau, une mesure de l'effet barrière, une connaissance de l'exposition de la peau à diverses conditions (soleil, pollution ou autre).

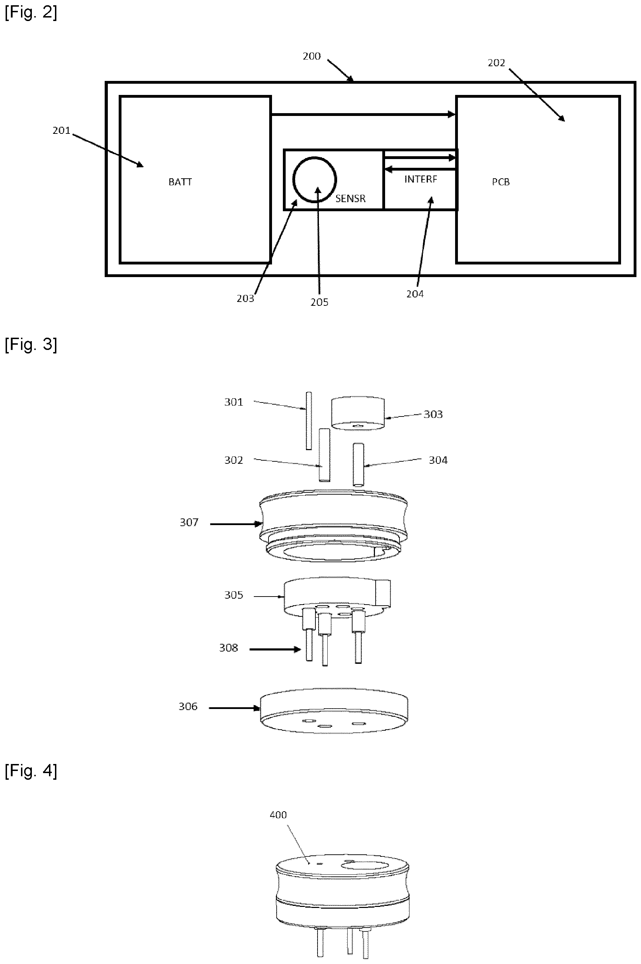

La **figure 2** illustre un dispositif **200** selon des modes de réalisation. Le dispositif **200** comporte une unité d'alimentation en énergie, par exemple une batterie **201** alimentant un circuit électronique **202.** Un capteur **203** est connecté au circuit **202** via une interface de connexion **204.**

Le capteur **203** est configuré pour recevoir le patch **205** tel que discuté ci-avant en référence à la **figure 1****.** Il est par ailleurs configuré pour transmettre des signaux électroniques, via l'interface **204,** au circuit électronique pour un traitement permettant ensuite la transmission de données de mesure à un dispositif distant, comme cela a été décrit ci-avant en référence à la **figure 1****.** Le capteur peut aussi être configuré pour recevoir directement une goutte comprenant des composés biologiques et/ou chimiques provenant de la couche superficielle de la peau d'un utilisateur ou directement une solution de principes actifs nécessaires à la formulation.

L'interface de connexion **204** permet en outre de connecter et de déconnecter le capteur **205** du circuit électronique **202.** Cela permet de remplacer le capteur de manière aisé et à la demande. Par exemple, l'interface comporte une connexion de type USB. D'autres types de connexion amovibles sont possibles.

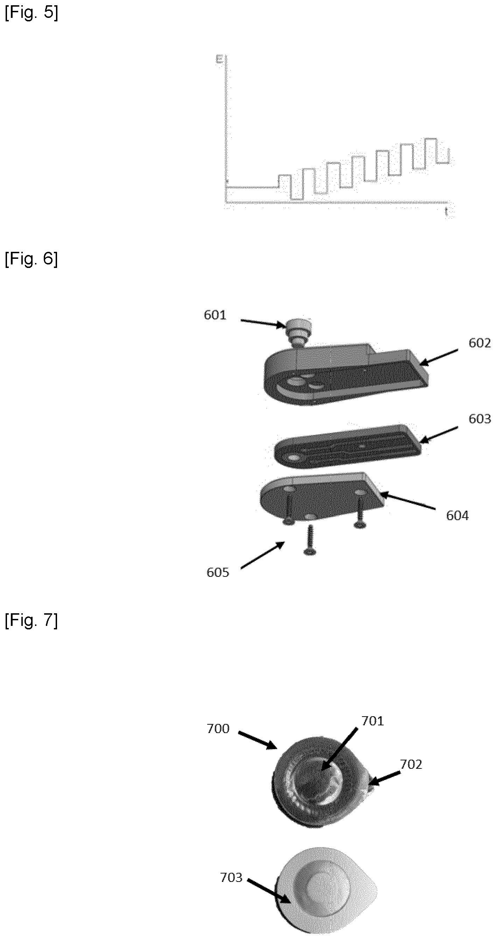

Le circuit électronique **202** comporte un potentiostat. Cela permet d'obtenir des mesures de qualité de manière répétée et répétable. Un potentiostat offre en outre l'avantage d'être de taille réduite et peu coûteux à produire industriellement. D'une manière générale, un potentiostat envoie un courant ou un potentiel et récupère un courant ou un potentiel. La façon dont il envoie le courant peut être soit linéaire, constante, en escalier etc. Selon des modes de réalisation, le potentiostat est agencé de manière à tenir dans un boîtier et à pouvoir s'interfacer avec d'une part le capteur, d'autre part, en communication sans fil, avec le dispositif distant. Les performances du potentiostat sont ajustables afin d'adapter au type de mesure (oxydation, réduction, nombre de cycles etc....) ou à des contraintes dermatologiques ou dermato-cosmétiques. De manière avantageuse, le potentiel peut être choisi sous forme de rampe en forme d'escalier comme illustré par la **figure 5****.** Le courant est par exemple mesuré avant et après chaque marche d'escalier en soustrayant avant/après pour supprimer les bruits et nettoyer le signal. Cela permet d'obtenir une courbe de différence de courant en fonction du potentiel.

Le dispositif **200** permet ainsi de réaliser une mesure de la santé de la peau de l'utilisateur de manière aussi efficace qu'en laboratoire. Comme cela est décrit dans ce qui suit, le capteur comporte un ensemble d'électrodes (non représentées) configurées pour entrer en contact avec le patch pré-imbibé et réaliser les mesures souhaitées (ou directement la goutte de solvant).

La **figure 3** est une vue éclatée d'un capteur selon des modes de réalisation.

Le capteur comporte un couvercle **306** configuré pour recevoir les broches de connexion **308** d'un connecteur **305.** Chaque broche est configurée pour connecter une électrode à l'interface de connexion du capteur pour transmettre les signaux électriques au circuit électronique, comme cela a été décrit en référence à la **figure 2****.** Le connecteur est configuré pour être fixé à une capsule **307** apte à recevoir les électrodes.

Le capteur peut comporter trois électrodes ou plus. Par exemple, les électrodes sont dans un matériau choisi parmi le carbone vitreux, l'argent massif et le graphite ou l'or. Par exemple, le capteur comporte une électrode de chaque matériau. Dans le mode de réalisation illustré, l'électrode **301** est en argent, l'électrode **303** est en carbone vitreux et l'électrode **302** en graphite. Un fil de cuivre **304** permet de connecter les électrodes aux broches du connecteur. L'utilisation d'au moins trois électrodes au lieu de deux usuellement permet de meilleurs résultats, notamment pour les mesures d'anti-oxydants, en particulier leur quantité.

La **figure 4** est une vie assemblée du capteur. Une fois assemblé, la surface **400** de la capsule est aplanie pour permettre de recevoir le patch (ou une goutte de solvant) et assurer un bon contact.

Des dimensions du capteur selon des modes de réalisation avec trois électrodes sont les suivantes. Le capteur peut avoir une forme globalement cylindrique, avec un diamètre de 10 mm. Le corps assemblé avec la capsule et le connecteur peut avoir une longueur de 20 à 30 mm (sans compter la longueur des broches de connexion **308**). Les électrodes peuvent avoir une longueur de 2 mm et être espacées, dans le plan **400** de la capsule par une distance de 3 mm deux à deux selon un triangle isocèle. Cette configuration permet un assemblage compact du capteur. En outre, elle assure la minimisation des interférences chimiques et électriques entre les électrodes, tout en contrôlant la quantité de liquide et nécessaire pour toucher les trois électrodes en même temps.

Selon des modes de réalisation, l'électrode en carbone vitreux a un diamètre assez élevé pour utiliser un courant électrique suffisant, mais un diamètre assez faible pour minimiser le bruit des électrodes. L'électrode en graphite a un diamètre par exemple au moins trois fois plus grand que celle en carbone vitreux pour assurer la stabilité des performances. L'électrode en argent ou or doit avoir un diamètre assez grand pour être manipulé pendant le processus d'intégration.

Des électrodes selon des modes de réalisation de l'invention permettent une mesure des biomarqueurs, notamment d'oxydo-réduction, fiable. Le montage du capteur est imperméable pour une compatibilité avec un usage avec un patch imbibé de solution électrolyte. La configuration du capteur est par ailleurs simple et fiable pour une industrialisation efficace.

Les matériaux choisis pour les électrodes permettent une utilisation répétée (capteur non jetable), avec une sensibilité et une précision suffisante. Par exemple, l'or, l'argent massif, le carbone vitreux massif et le graphite massif permettent une durabilité avec une utilisation jusqu'à 100 000 mesures. Ces matériaux permettent aussi de recycler le capteur.

Les matériaux évoqués peuvent être régulièrement polis (par exemple en frottant doucement un écouvillon imbibé d'eau ou de solution d'alumine et en rinçant). Cela permet la régénération de la surface des électrodes en éliminant les impuretés et le maintien de la qualité du signal.

La fiabilité du capteur est accrue par rapport aux encres ou aux dépôts habituellement utilisés dans les électrodes du marché mais réputés moins résistants aux contraintes mécaniques et chimiques. Ce type d'électrodes est jetable, à base d'encre métallique et a un temps d'utilisation très court et doit être remplacé après quelques utilisations (moins de 10).

La capsule du capteur peut par exemple être faite en polymère étanche afin de permettre une utilisation dans des conditions humides telles que les salles de bains ou les conditions climatiques asiatiques. Les résultats obtenus au moyen du capteur ne dépendent ainsi pas de l'hygrométrie.

La **figure 6** illustre une interface de connexion du capteur à un circuit électronique, comme cela a été décrit en référence à la **figure 2****.**

Le capteur **601** est inséré dans une capsule supérieure **602** comprenant des orifices pour le passage des broches de connexion du capteur. La capsule supérieure est en outre configurée pour recevoir un circuit électronique de connexion **603**, par exemple de type PCB. Le circuit électronique de connexion **603** comporte des pistes de conduction électriques configurées pour entrer en contacts respectifs avec les broches de connexion du capteur. L'interface de connexion comporte enfin une capsule inférieure **604** configurée pour fixer le circuit électronique de connexion entre elle et la capsule supérieure, par exemple au moyen de trois vis. D'autres modes de fixation sont possibles en alternative aux vis. Le matériau des capsules supérieure et inférieur peut être choisi pour assurer l'étanchéité.

Selon des modes de réalisation, le circuit électronique de connexion 6**03** comporte à son extrémité opposée à celle qui est en contact avec les broches de connexion du capteur une prise mâle (par exemple de type USB). Cette prise mâle est configurée pour être connectée à une prise femelle (non représentée) présente sur le circuit électronique auquel doit être interfacé le capteur.

Selon des modes de réalisation, la capsule supérieure peut en outre comporter un joint torique (non représenté) autour du capteur pour assurer l'étanchéité lorsqu'un patch imbibé ou une goutte de solvant est déposé(e) sur celui-ci. Par exemple, le joint a une dureté entre 50 et 60 shore. L'interface peut en outre comporter un élément de fixation dans le dispositif selon des modes de réalisation ou alternativement sur le circuit électronique avec lequel s'interface le capteur.

La **figure 7** illustre un dispositif de prélèvement selon des modes de réalisation. Par exemple, il s'agit d'un patch.

Le patch **700** a une forme sensiblement en poire, avec en son centre un logement pour recevoir une ouate imbibée **701.** Le patch est recouvert par un opercule qui peut se retirer pour dévoiler la ouate imbibée. La forme en poire permet la manipulation de l'opercule, en particulier la pointe **702** de la poire permet à l'utilisateur de se saisir de l'opercule pour le retirer.

De manière avantageuse, la face du patch recevant l'opercule peut avoir une surface adhésive **703** pour adhérer à l'opercule lorsque celui-ci recouvre la ouate imbibée mais aussi pour accrocher au dispositif, lorsque le patch est placé sur le dispositif.

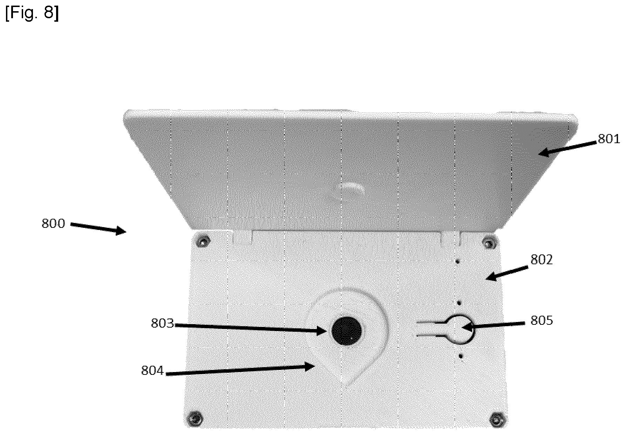

La **figure 8** illustre un dispositif selon un mode de réalisation. Par exemple, le dispositif se présente sous la forme d'un boîtier **800**, par exemple de forme globalement parallélépipèdique. Le boîtier comporte un corps de boîtier **802** pour loger les éléments décrits ci-avant (par exemple en référence à la **figure 2**)**.** Le boîtier peut être refermé par un couvercle **801.** Dans le corps de boitier, un capteur **903** émerge au niveau d'un creux **804** de la forme d'un patch, tel que par exemple décrit en référence à la **figure 7****.** Le boîtier peut en outre comporter une interface utilisateur **805**, tel qu'un bouton pressoir par exemple.

La présente invention a été décrite et illustrée dans la présente description détaillée en référence aux figures jointes. Toutefois la présente invention ne se limite pas aux formes de réalisation présentées. D'autres variantes, modes de réalisation et combinaisons de caractéristiques peuvent être déduits et mis en oeuvre par la personne du métier à la lecture de la présente description et des figures annexées.

Pour satisfaire des besoins spécifiques, une personne compétente dans le domaine de l'invention pourra appliquer des modifications ou adaptations.

Dans les revendications, le terme *"comporter"* n'exclut pas d'autres éléments ou d'autres étapes. Les différentes caractéristiques présentées et/ou revendiquées peuvent être avantageusement combinées. Leur présence dans la description ou dans des revendications dépendantes différentes, n'exclut pas en effet la possibilité de les combiner. Les signes de référence ne sauraient être compris comme limitant la portée de l'invention.

## Revendications

1. Dispositif (200, 900) de mesure de paramètres biologiques ou chimiques relatifs à la peau d'un utilisateur ou à l'efficacité dermo-cosmétique et dermatologique d'une formule de soin de la peau comprenant :
un capteur (203, 903) configuré pour réaliser une mesure à partir d'une goutte d'une solution électrolyte comprenant des composés biologiques et/ou chimiques ou à partir d'une ouate imbibée (701) d'une telle solution électrolyte,
un circuit électronique (202) configuré pour recevoir des signaux électriques dudit capteur (203, 803) et pour réaliser un traitement en vue de transmettre des données de mesures à un dispositif distant (104),
un logement (804) configuré pour recevoir ladite goutte et/ou un dispositif de prélèvement (700) comprenant ladite ouate imbibée (700).

2. Dispositif selon la revendication 1, dans lequel lesdits composés biologiques et/ou chimiques provenant d'au moins l'un parmi la couche superficielle de la peau d'un utilisateur, de principes actifs ou de formules de soin de peau dermatologiques ou dermo- cosmétiques.

3. Dispositif selon la revendication 1, dans lequel ladite mesure comporte au moins l'un parmi la détection de la présence de biomarqueurs de la peau dudit utilisateur et la détermination de l'la nature des anti-oxydants d'un composé destiné à être appliqué sur la peau dudit utilisateur.

4. Dispositif selon la revendication 1, dans lequel ledit circuit électronique est en outre configuré pour transmettre lesdites données de mesure audit dispositif distant (104).

5. Dispositif selon l'une quelconque des revendications précédentes, comprenant en outre une interface de connexion (204) pour connecter ledit capteur (203, 803) audit circuit électronique (202), ladite connexion étant configurée pour connecter et déconnecter ledit capteur à la demande, de manière amovible.

6. Dispositif selon l'une quelconque des revendications précédentes, dans lequel ledit circuit électronique comporte un potentiostat, le potentiel dudit potentiostat étant analysé sous forme de rampe en escalier.

7. Dispositif selon l'une quelconque des revendications précédentes, dans lequel ledit capteur comporte au moins trois électrodes (301, 302, 303) dont au moins l'une est formée d'un matériau choisi parmi le graphite, l'argent massif, le carbone vitreux, et l'or.

8. Dispositif selon la revendication 6, comprenant trois électrodes et dans lequel, lesdites électrodes affleurent dans un même plan de contact (400) avec la ouate imbibée (701) ou la goutte et sont distribuées selon les sommets d'un triangle isocèle dans ledit plan (400).

9. Système de mesure de paramètres relatifs à la peau d'un utilisateur comprenant :
un dispositif (200, 800) selon l'une quelconque des revendications précédentes, et
un dispositif de prélèvement (800) comprenant une ouate imbibée (701) d'une solution électrolyte comprenant des composés biologiques et/ou chimiques.

10. Système de mesure selon la revendication 9, dans lequel lesdits composés biologiques et/ou chimiques proviennent d'au moins l'un parmi la couche superficielle de la peau d'un utilisateur ou de formules de soin de peau ou d'actifs dermatologiques ou dermo- cosmétiques.

11. Système selon la revendication 9 ou 10, dans lequel ledit dispositif de prélèvement a une forme en poire, ladite ouate imbibée (701) étant placée en son centre et un opercule recouvrant ladite ouate imbibée.

12. Système selon l'une des revendications 9 à 11, comprenant en outre un dispositif distant (104) configuré pour recevoir des données de mesure dudit dispositif de prélèvement (700) et pour réaliser au moins un traitement à partir de ces données.

13. Système selon la revendication 11, comprenant outre au moins un serveur distant (105, 107), configuré pour communiquer directement ou indirectement avec un dispositif distant (104) et pour stocker des données d'utilisateur issues de mesures de paramètres relatifs à leur peau |
